(19) Europäisches Patentamt
European Patent Office
Office européen des brevets

(11) Publication number: **0 521 420 A1**

# (12) EUROPEAN PATENT APPLICATION

(21) Application number: **92110913.8**

(22) Date of filing: **27.06.92**

(51) Int. Cl.5: **C07C 43/225**, C09K 19/30, G02F 1/13

(30) Priority: **04.07.91 EP 91111095**

(43) Date of publication of application: **07.01.93 Bulletin 93/01**

(84) Designated Contracting States: **DE GB**

(71) Applicant: **MERCK PATENT GESELLSCHAFT MIT BESCHRÄNKTER HAFTUNG**
**Frankfurter Strasse 250**
**W-6100 Darmstadt(DE)**

(72) Inventor: **Hittich, Reinhard, Dr.**
**Am Kirchberg 11**
**W-6101 Modautal 1(DE)**
Inventor: **Coates, David, Dr.**
**87, Sopwith Crescent, Merley**
**Wimborne, Dorset BH21 3SW(GB)**
Inventor: **Greenfield, Simon, Dr.**
**2, Blackbird Close**
**Creekmoor, Poole, BH17 7YA(GB)**

## (54) Fluorinated cyclohexyl derivatives.

(57) The invention relates to fluorinated cyclohexyl derivatives of formula I

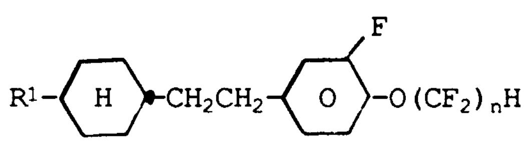

I

wherein

$R^1$ denotes an alkyl residue with up to 12 carbon atoms wherein one or two non-adjacent $CH_2$-groups may also be replaced by -O-, -O-CO-, -CO-O- and/or -CH=CH-, and

n is 1 or 2,

and also to liquid crystalline media being a mixture of at least 2 compounds, characterized in that at least one compound is a fluorinated cyclohexyl derivative according to formula I.

EP 0 521 420 A1

The invention relates to fluorinated cyclohexyl derivatives of formula I,

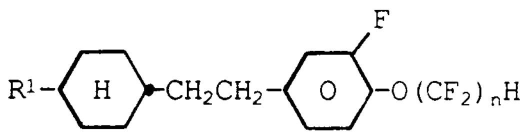

I

wherein

$R^1$ denotes an alkyl residue with up to 12 carbon atoms wherein one or two non-adjacent $CH_2$-groups may also be replaced by -O-, -O-CO-, -CO-O- and/or -CH = CH-, and

n is 1 or 2,

and also to liquid crystalline media being a mixture of at least 2 compounds, characterized in that at least one compound is a fluorinated cyclohexyl derivative according to formula I.

The invention was based on the object of discovering new stable liquid crystal or mesogenic compounds which are suitable as components of liquid crystalline media and, in particular, have advantageous values for optical and dielectric anisotropy combined with very favorable elastic properties, low viscosity and high nematogenity.

Similar cyclohexyl derivatives are described in WO 91/03450:

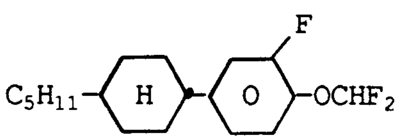

K 9 I

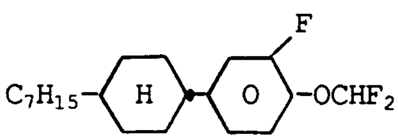

K 7 N (-28) I

These compounds, however, still pose problems in the mixture development due to the low clearing point.

It has now been found that fluorinated cyclohexyls of formula I are highly suitable as components of liquid crystalline media. In particular, they have especially advantageous values of optical and dielectric anisotropy ($\Delta\epsilon$ $\epsilon\perp$ and $\epsilon\|$) and unusually high values for the elastic constants. It is also possible to obtain stable liquid crystal phases with a broad nematic mesophase range including a good deep timperature behaviour and a comparatively low viscosity with the aid of these compounds. Such phases are of particular interest for supertwisted nematic displays.

The compounds of the formula I are colourless in the pure state and are liquid crystalline in a temperature range which is favourably placed for electrooptical use. They are very stable towards chemicals, heat and light.

The invention thus relates to the fluorinated cyclohexyl derivatives of the formula I, to liquid crystalline media with at least two liquid crystalline components, wherein at least on component is a compound of the formulae I and to liquid crystal display devices containing such media.

Above and below, $R^1$ and n have the meaning given unless expressly indicated otherwise.

$R^1$ is preferably alkyl, alkoxy, oxaalkyl, alkanoyloxy or alkenyl and can exhibit a straight-chain or branched structure.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hoxoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxypropyl (= methoxymethyl), 2-(=ethoxymethyl) or 3-oxybutyl (= 2-methoxyethyl), 2-, 3- or 4-oxypentyl, 2-, 3-. 4- or 5-oxyhexyl, 2-, 3-, 4- 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-, 7-, 8-, or 9-oxadexyl.

Alkenyl is preferably straight-chain and has 2 or 10 C atoms.

It is accordingly, in particular, vinyl, prop-1- or prop-2-e-nyl, but-1-, -2- or -3-enyl, pent-1-, -2-, -3- or -4-enyl, hex-1-, -2-, -3-, -4- or -5-enyl, hept-1-, -2-, -3-, -4-, -5-or -6-enyl, oct-1-, -2-, -3-, -4-, -5- -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I containing a branched terminal group can occasionally be or importance because of an improved solubility in the customary liquid crystal base materials, but in particular as chiral doping substances if they are optically active.

Branched groups of this type as a rule contain not more than one chain branching. Preferred branched radicals are isopropyl, 2-butyl (= 1-methylpropyl), isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methylpropoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (=2-octyloxy), 2-oxa-3-methylbutyl, 3-oxy-4-methylpentyl, 4-methylhexyl, 2-nonyl, 2-decyl, 2-dodecyl, 6-methyloctoxy, 6-methyloctanoyloxy, 4-methylheptyloxycarbonyl, 2-methylbutyryloxy, 3-methylvaleryloxy, 4-methylhexanoyloxy, 2-methyl-3-oxapentyl and 2-methyl-3-oxahexyl.

In the case of compounds with a branched terminal group $R^1$, formula I includes both the optical antipodes and racemates as well as mixtures thereof.

n is preferably 1.

Of the compounds of the formula I and subformulae thereof, those in which at least one of the radicals contained therein has one of the preferred meanings given are preferred.

The compounds of the formula I are prepared by methods which are known per se, sich as are described in the literature (for example in the standard works, such as Houben-Weyl, Methoden der Organischen Chemie [Methods of Organic Chemistry], Georg Thieme Verlag, Stuttgart), and in particular under reaction conditions which are known and suitable for the reactions mentioned. Variants which are known per se and are not mentioned in more detail here can also be used in this connection.

If desired, the starting materials can also be formed in situ, such that they are not isolated from the reaction mixture but are immediately reacted further to give the compounds of the formula I.

A preferred route for preparation starts from the easily accessible phenol of the formula II

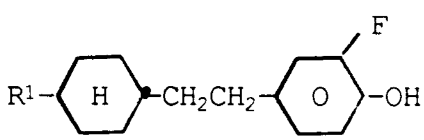

which can be obtained according to the following route:
The route to phenol II is

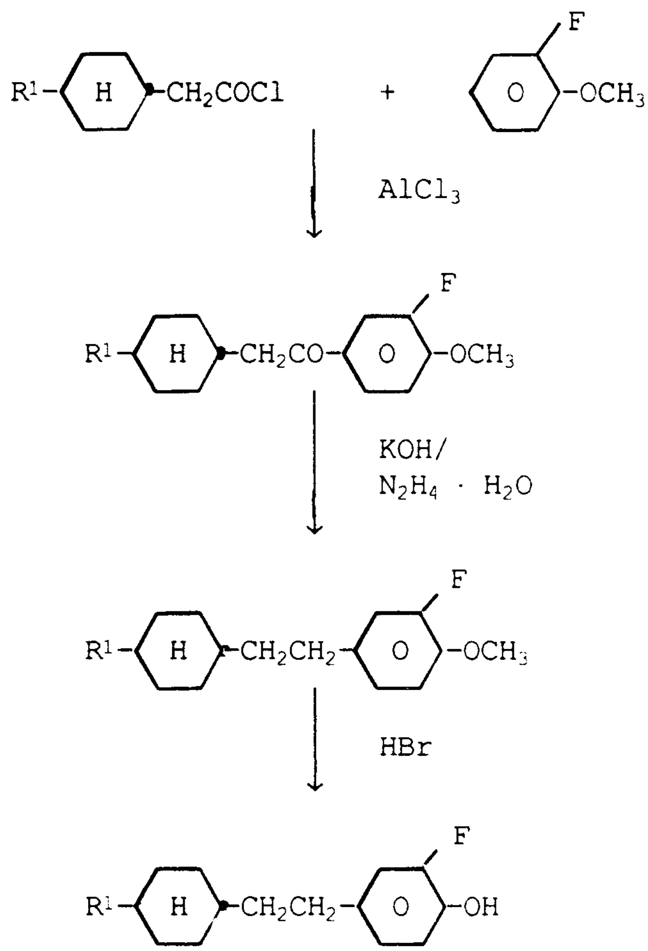


The compounds of the formula I are prepared from the precursor II by reacting with chlorodifluoromethane or tetrafluorethylene respectively in the presence of a base.

An alternative route is shown in Scheme 1:

Scheme 1

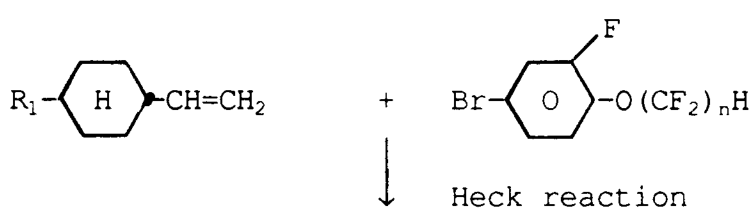

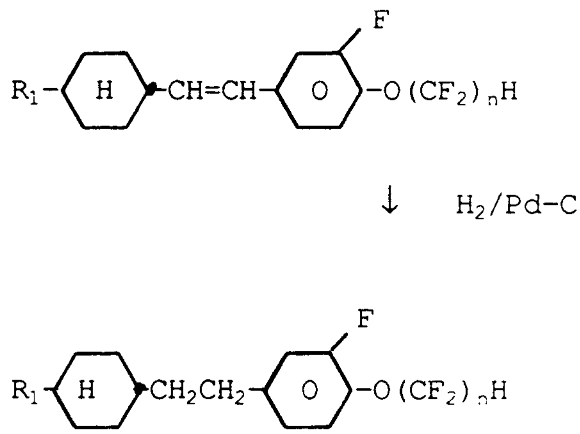


Other routes areapparent to the skilled worker. In addition it is possible to follow the above shown routes with a group X being different from the desired one and to introduce the desired group X in the final step, e.g. conversion of -OMe to -OH and finally to $OCF_2CF_2H$ or $-OCHF_2$. All these steps and the corresponding reaction conditions are known to the skilled worker.

In addition to one or more compounds for formula I the liquid crystal media according to the invention preferably contain 2-40 components and in particular 4-30 components. Liquid crystal media being composed of one or more compounds of formula I 7-25 other components are especially preferred.

These additional components are preferably chosen from the nematic (monotropic or isotropic) substances; in particular from the classes of azoxybenzenes, benzylideneanilines, biphenyls, terphenyls, phenyl or cyclohexyl benzoates, phenyl or cyclohexyl cyclohexynecarboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexynecarboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexyldioxanes, phenyl-or caclohexyl-1,3-dithianes, 1,2-diphenylethanes, 1,2-dicyclohexylethanes, 1-phenyl-2-cyclohexylethanes, 1-cyclohexyl-2-(4-phenyl-cyclohexyl)-ethanes, 1-cyclohexyl-2-biphenylethanes, 1-phenyl-2-cyclohexylphenylethanes, optionally halogenated stilbenes, benzyl phenyl ethers, tolanes and substituted cinnamic acids.

The 1,4-phenylene groups of these compounds may be fluorinated.

The most important compounds which ar epossible constituents of liquid crystal media according to the invention can be characterized by the formulae 1, 2, 3, 4 and 5:

R'-L-U-R" 1

R'-L-COO-U-R" 2

R'-L-OOC-U-R" 3

R'-L-$CH_2CH_2$-U-R" 4

R'-L-C≡C-U-R" 5

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residue Phe denotes unsubstituted or fluorinated 1,4-phenylen, Cyc trans- 1,4-cyclohexylene or 1,4-cyclohexylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid

crystal media further containing at the same time one or more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R' and R'' are independently from each other alkyl, alkenyl, alkoxy, alkenoxy with up tp 8 carbon atoms. R' and R'' differ from one another in most of these compounds, one of the residues usually being alkyl or alkenyl. In another preferred subgroup of the compounds of formulae 1, 2, 3, 4 and 5 (subgroup 2) R'' denotes -CN, -$CF_3$, -F, -Cl or -CS while R' has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal media according to the invention preferably contain in addition to components selected from subgroup 1 also components of subgroup 2, the percentage of these components being as follows:

| | |
|---|---|
| subgroup 1: | 20 to 90 %, in particular 30 to 90 % |
| subgroup 2: | 10 to 50 %, in partiuclar 10 to 50 % |

In these liquid crystal media the percentages of the compounds according to the invention and the compounds of subgroup 1 and 2 may add up to give 100 %.

The media according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

The media according to the invention are prepared in a manner which is customary per se. As a rule, the components are dissolved in one another, advantageously at elevated temperature. The liquid crystal media according to the invention can be modified by suitable additives so that they can be used in all the types of liquid crystal display devices. Such additives are known to the expert and are described in detail in the literature (H. Kelker/R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

In the present application and in the examples below, the structures of the liquid-crystal compounds are indicated by acronyms, with the transformation into chemical formulae taking place in accordance with Tables A and B below. All radicals $C_nH_{2n+1}$ are straight-chain alkyl radicals containing n and/or m carbon atoms. The coding in Table B is self-evident. In Table A, only the acronym for the base structure is given. In individual cases, the acronym for the base structure is followed, separated by a hyphen, by a code for the substituents $R^1$, $R^2$, $L^1$, $L^2$ and $L^3$.

| Code für $R^1$, $R^2$, $L^1$, $L^2$ | $R^1$ | $R^2$ | $L^1$ | $L^2$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | H | F |
| nF | $C_nH_{2n+1}$ | F | H | H |
| nOF | $OC_nH_{2n+1}$ | F | H | H |
| nCl | $C_nH_{2n+1}$ | Cl | H | H |
| nF.F | $C_nH_{2n+1}$ | F | H | F |
| nOmFF | $C_nH_{2n+1}$ | $OC_mH_{2m+1}$ | F | F |
| nmF | $C_nH_{2n+1}$ | $C_mH_{2m+1}$ | F | H |
| $nCF_3$ | $C_nH_{2n+1}$ | $CF_3$ | H | H |
| $nOCF_3$ | $C_nH_{2n+1}$ | $OCF_3$ | H | H |
| $nOCF_2$ | $C_nH_{2n+1}$ | $OCHF_2$ | H | H |
| nS | $C_nH_{2n+1}$ | NCS | H | H |
| rVsN | $C_rH_{2r+1}$-CH = CH-$C_sH_{2s}$- | CN | H | H |
| rEsN | $C_rH_{2r+1}$-O-$C_sH_{2s}$- | CN | H | H |
| nNF | $C_nH_{2n+1}$ | CN | F | H |
| nAm | $C_nH_{2n+1}$ | $COOC_mH_{2m+1}$ | H | H |

**Tabelle A:**

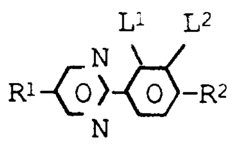

**PYP**

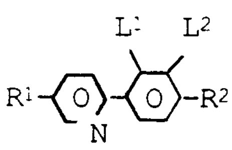

**PYRP**

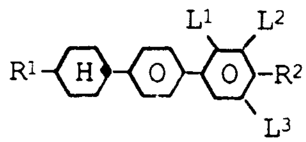

BCH

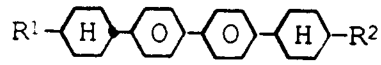

CBC

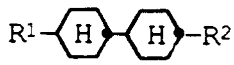

CCH

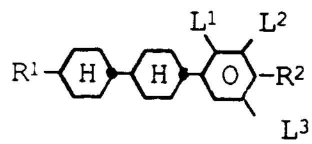

CCP

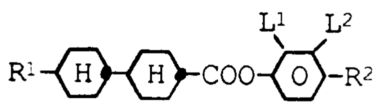

CP

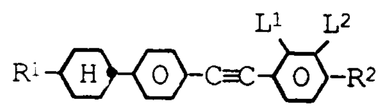

CPTP

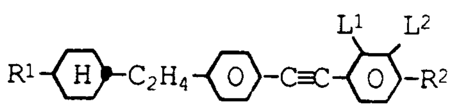

CEPTP

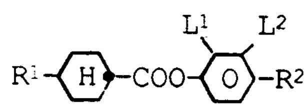

D

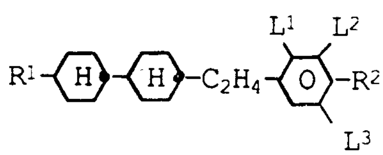

ECCP

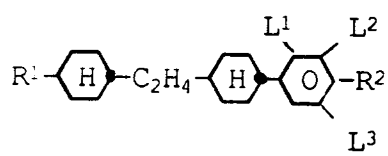

CECP

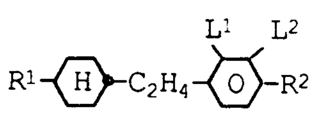

EPCH

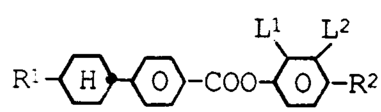

HP

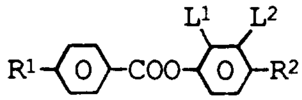

**ME**

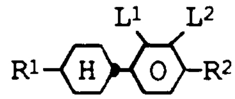

**PCH**

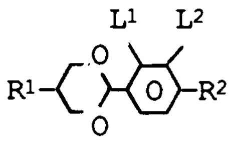

**PDX**

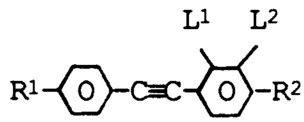

**PTP**

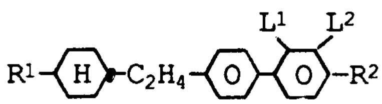

**BECH**

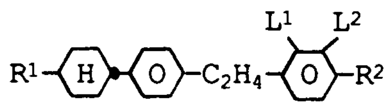

**EBCH**

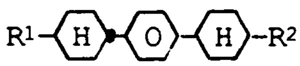

**CPC**

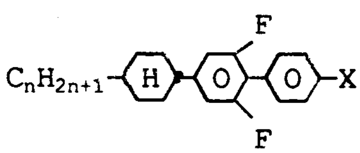

**CUP-nX**

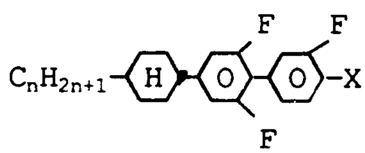

**CUP-nX.F**

**Tabelle B:**

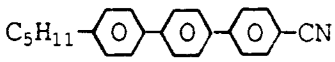

**T15**

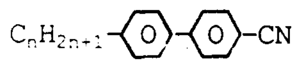

**K3n**

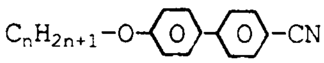

**M3n**

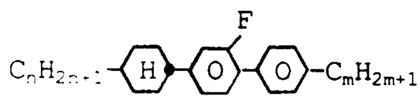

**BCH-n.Fm**

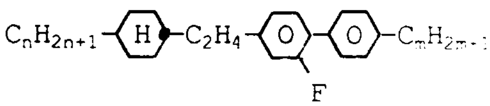

**Inm**

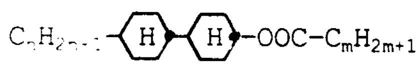

**C-nm**

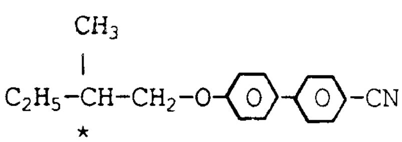

**C15**

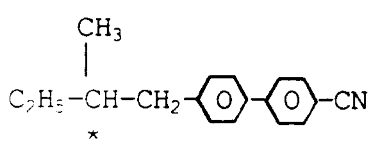

**CB15**

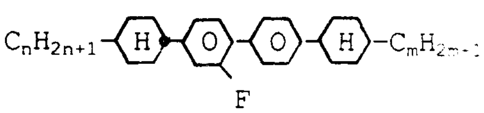

**CBC-nmF**

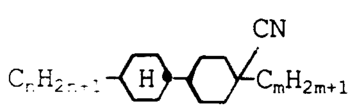

**CCN-nm**

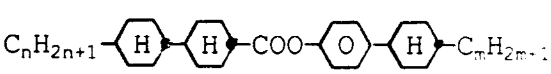

**CCPC-nm**

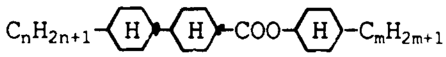

**CH-nm**

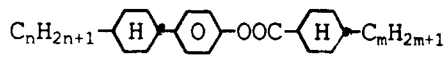

**HD-nm**

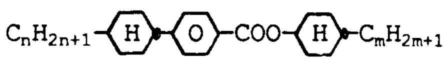

**HH-nm**

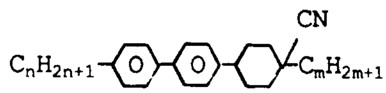

**NCB-nm**

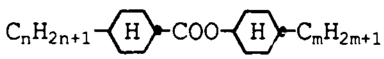

**OS-nm**

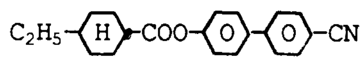

**CHE**

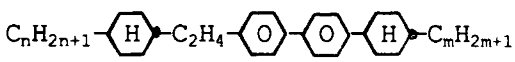

**ECBC-nm**

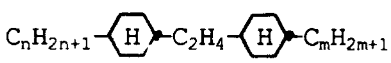

**CCH-nm**

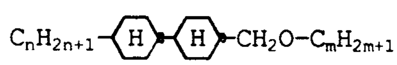

**CCH-n1EM**

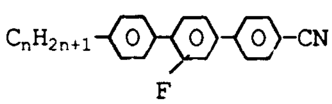

**T-nFn**

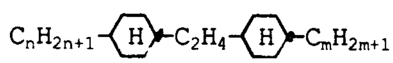

**ECCH-nm**

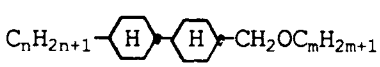

**CCH-n1Em**

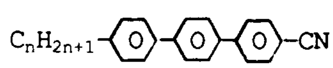

**T-nFn**

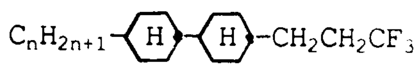

**CCH-n2CF$_3$**

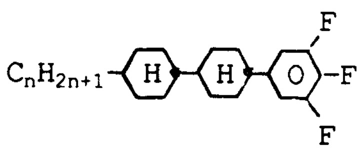

**CCP-nF.F.F.**

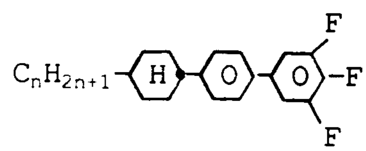

**BCH-nF.F.F**

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is separated off, dried and evaporated, and the product is purified by crystallization and/or chromatography.

Further are:

C: crystalline-solid state, S: smectic phase (the index denoting the type of smectic phase), N: nematic phase, CH: cholesteric phase, I: isotropic phase. The number being embraced by 2 of these symbols denotes the temperature of phase change.

Examples for production

Example 1

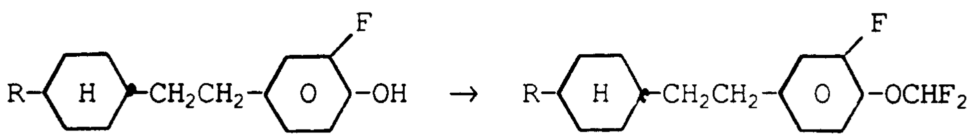

(I)

The fluoro phenyl (R = $C_5H_{11}$, 29.2 g, 0.1 mol) is dissolved in a mixture of dioxan (40 ml), water (25 ml) and NaOH (20.0 g, 0.5 mol) and the solution heated to 70°. Chlorodifluoromethane is bubbled through the stirred reaction mixture until no more gas is absorbed and tlc analysis shows an absence of phenol. The cooled reaction mixture is diluted with water (100 ml) and extracted with dichloromethane (3 x 50 ml), the extracts are washed with water, dried and the solvent distilled off. The crude product is purified by distillation (and/or recrystallization). $\Delta\epsilon$ = 6.07, $\Delta n$ = 0.044

The following compounds are obtained analogously or by reaction of II with tetrafluoroethene:

| $R^1$ | n ethyl 1 |
|---|---|
| n-propyl | 1 |
| n-butyl | 1 |
| n-hexyl | 1 |
| n-heptyl | 1 |
| ethyl | 2 |
| n-propyl | 2 |
| n-butyl | 2 |
| n-pentyl | 2 |
| n-hexyl | 2 |
| n-heptyl | 2 |

Examples of mixtures

Example A

A liquid crystalline mixture consisting of

| | |
|---|---|
| 10,0 % | EPCH-5OCF$_2$.F |
| 8,0 % | EPCH-6OCF$_2$.F |
| 6,0 % | EPCH-7OCF$_2$.F |
| 8,0 % | CCP-2OCF$_3$ |
| 12,0 % | CCP-3OCF$_3$ |
| 9,0 % | CCP-4OCF$_3$ |
| 9,0 % | CCP-5OCF$_3$ |
| 12,0 % | BCH-3F.F |
| 10,0 % | BCH-5F.F |
| 5,0 % | ECCP-3OCF$_3$ |
| 5,0 % | ECCP-5OCF$_3$ |
| 2,0 % | CBC-33F |
| 2,0 % | CBC-53F |
| 2,0 % | CBC-55F |

exhibits a high clearing point, a low optical anisotropy and a high value for $\Delta\epsilon$.

**Claims**

**1.** Fluorinated cyclohexyl derivatives of formula I wherein

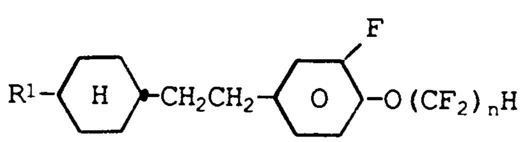

I

$R^1$ denotes an alkyl residue with up to 12 carbon atoms wherein one or two non-adjacent $CH_2$-groups may also be replaced by -O-, -O-CO-, -CO-O- and/or -CH=CH-, and

n is 1 or 2.

**2.** Liquid crystalline medium being a mixture of at least two compounds, characterized in that at least one compound is a fluorinated cyclohexyl derivative according to claim 1.

**3.** Liquid crystal display device, characterized in that it contains a liquid crystalline medium according to claim 2.

**4.** Electrooptical display device, characterized in that it contains a liquid crystalline medium according to claim 2.

European Patent Office

# EUROPEAN SEARCH REPORT

Application Number

EP 92 11 0913

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | WO-A-9 001 056 (MERCK PATENT GMBH)<br>* page 46; claim 1 *<br>--- | 1-4 | C07C43/225<br>C09K19/30<br>G02F1/13 |
| Y | WO-A-8 808 441 (MERCK PATENT GMBH)<br>* page 63; claim 1 *<br>--- | 1-4 | |
| Y | WO-A-9 015 115 (MERCK PATENT GMBH)<br>* page 20; claim 1 *<br>--- | 1-4 | |
| Y | WO-A-9 014 406 (MERCK PATENT GMBH)<br>* the whole document *<br>--- | 1-4 | |
| P,X | WO-A-9 115 450 (MERCK PATENT GMBH)<br>* page 21 - page 28; claim 1 *<br>--- | 1-4 | |
| P,X | WO-A-9 116 398 (MERCK PATENT GMBH)<br>* the whole document *<br>----- | 1-4 | |

| TECHNICAL FIELDS SEARCHED (Int. Cl.5 ) |
|---|
| C07C<br>C09K |

The present search report has been drawn up for all claims

| Place of search | Date of completion of the search | Examiner |
|---|---|---|
| THE HAGUE | 08 SEPTEMBER 1992 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document